# EUROPEAN PATENT APPLICATION

(11) **EP 2 192 119 A1**
(43) Date of publication of application: **02.06.2010**
(21) Application number: 08170115.3
(22) Date of filing: 27.11.2008
(51) Int. Cl.: C07D 307/89, B27K 3/34

(54) **Wood preservation agent**

(71) Applicant: Stichting Dienst Landbouwkundig Onderzoek, 6701 BH Wageningen (NL)
(72) Inventor: Blaauw, Roelf, 6708 MD Wageningen (NL); van Haveren, Jacobus, 6717 XB Ede (NL); van Loo, Eibertus Nicolaas, 6708 SL Wageningen (NL)
(74) Representative: Hatzmann, Martin

(57) **Abstract**

Disclosed is a method for the preservation of wood comprising the impregnation of wood, and heating, with a liquid based on a natural oil comprising a conjugated polyunsaturated fatty acid. Preferred are oils comprising a multiple conjugated polyunsaturated fatty acid, such as calendula oil or tung oil. The liquid can comprise the oil itself, in an unmodified form, or it can comprise a modification thereof in the form of a Diels-Alder adduct with a dienophile such as maleic anhydride.

## Description

### Field of the Invention

The invention pertains to a method for the preservation of wood comprising impregnating wood with a wood preservation agent in the form of a natural oil-based liquid comprising a polyunsaturated fatty acid, and heating the impregnated wood. The invention also pertains to a chemically modified natural oil. The invention further pertains to the use of conjugated polyunsaturated natural oils, and modifications thereof, in the preservation of wood, and to wood thus preserved.

### Background of the Invention

In the timber industry efforts are ongoing to make wood more durable. This refers, *inter alia* to the desire that wood is better capable of withstanding the influence of moisture, which is due to lead to dimensional instability and wood rot. The provision of more durable wood is of particular importance in order to be able to replace tropical hardwood by ecologically better acceptable wood, such as spruce or pine, which by nature are less durable.

One of these efforts is disclosed by B.F. Tjeerdsma et al. in Proceedings of the Second European Conference on Wood Modification (2005). Herein oil heat treatments are applied, using rapeseed oil, linseed oil, and a modified, maleinized linseed oil. The latter is also disclosed in EP 1 174 231, to which Tjeerdsma et al. refer. The modified oil is made by maleinizing the oil in a reaction sequence which in itself is well-known in the field of organic chemistry, viz. an "ene" reaction, followed by a Diels-Alder cyclo-addition.

For completeness' sake, reference is made to the existence of so-called drying oils, which usually comprise polyunsaturated fatty acids and which find use, *inter alia,* as or in wood varnish. Such drying oils are applied, and allowed to harden (the "drying"), and are then capable of providing some protection to outside influences, much like a paint. The method to which the invention pertains, different from paints and other wood finishes, is directed to providing a wood that is essentially modified by reaction with the wood preservation agent. This is generally accomplished in a reaction vessel by the combination of the impregnation and heating steps.

Whilst the results as presented strongly favor the use of the modified oil, the latter is associated with drawbacks that render its practical use more difficult. Particularly, in preparing the modified oil a very high temperature (200°C) is required. Also, said preparation process turned out to be difficult to reproduce.

It is thus desired to provide a method of the above-indicated type in which a good wood preservation can be obtained, without unfeasible chemical modification of the oil. It is also desired to provide a possibility for a better feasible chemical modification, and to therewith yield a better wood preservation.

### Summary of the Invention

In order to better address one or more of the foregoing desires, the invention, in one aspect, is a method for the preservation of wood, comprising impregnating the wood with a preservation agent in the form of a natural oil-based liquid comprising a polyunsaturated fatty acid, and heating the impregnated wood, wherein the natural oil comprises a conjugated polyunsaturated fatty acid, and particularly a polyunsaturated fatty acid containing more than two conjugated double bonds (hereinafter also referred to as "multiple conjugated polyunsaturated fatty acid" or abbreviated as MCPFA).

In another aspect, the invention is a modified natural oil obtainable by the reaction of a dienophile, such as maleic anhydride, with a natural oil comprising a conjugated polyunsaturated fatty acid.

In yet another aspect, the invention resides in the use of a natural oil comprising a conjugated polyunsaturated fatty acid in the preservation of wood, wherein the oil is used either unmodified, or modified by reaction with a dienophile such as maleic anhydride.

In still a further aspect, the invention pertains to wood modified by chemical reaction with a preserving agent obtainable from a natural oil comprising a conjugated polyunsaturated fatty acid.

### Detailed Description of the Invention

In its most simple, and already therefore advantageous form, the invention puts to use an unmodified natural oil, selected from those oils that comprise one or more conjugated polyunsaturated fatty acids, and particularly one or more multiple conjugated polyunsaturated fatty acids, and mixtures thereof. It will be understood that the invention can also be carried out using synthetic MCPFA's, such as dehydrated castor oil. Other examples of synthetically obtained conjugated fatty acid (esters) include conjugated linoleic acids and conjugated linolenic acids.

The term "conjugated" in connection with a multiple unsaturation is known to the skilled person in organic chemistry, and refers to the presence of two or more carbon-carbon double bonds (i.e. C=C bonds) that are covalently linked to each other via a single carbon-carbon bond. Or, in other words, both carbon atoms of a single covalent carbon-carbon bond are covalently attached to a further carbon atom through a double bond. A conjugated system, in its simplest form, can be depicted as -C=C-C=C-.

Natural oils generally comprise a mixture of fatty acids, including unsaturated or polyunsaturated fatty acids. In the present invention, natural oils are selected that comprise a conjugated polyunsaturated fatty acid. These oils are known and include calendula oil, tung oil, pomegranate seed oil, dimorphoteca seed oil and oiticica oil.

E.g. tung oil comprises in its fatty acid composition over 60 wt.% of eleostearic acid, i.e. (9E,11E,13Z)-octadeca-9,11,13-trienoic acid, which has three conjugated double bonds. Calendula oil comprises 50-60% of α-calendic acid, which is (8E,10E,12Z)-octadeca-8,10,12-trienoic acid, i.e. also a polyunsaturated fatty acid having three conjugated double bonds. Pomegranate seed oil comprises about 65 wt.% of (9Z,11E,13Z)-octadecatrienoic acid (punicic acid). Dimorphoteca seed oil comprises about 60 wt.% of 9-hydroxy-(10E,12Z)-octadecadienoic acid (dimorphecolic acid). Oiticica oil comprises about 78 wt.% of 4-oxo-eleostearic acid (licanic acid).

In fact, it is preferred in the invention to make use of natural oils comprising a triple conjugated unsaturated fatty acid, such as in calendula oil or tung oil (i.e. having three conjugated double bonds, for which a simplified formula is -C=C-C=C-C=C-). The oil preferably is selected from the group consisting of calendula oil, tung oil, and mixtures thereof.

The multiple, preferably triple, conjugated fatty acid-containing oils can be put to use in several ways.

In one embodiment, the natural oil-based liquid comprises the oil itself, i.e. the oil is used in an unmodified form. This embodiment is advantageous *inter alia* in that it does not require conducting a chemical reaction prior to providing the wood preservation agent, and thus provides an easy to conduct, straightforward wood preservation method. This method comprises the aforementioned impregnation and heating steps. Herein the oil can be used unheated for impregnation, after which excess oil is removed and the impregnated wood is heated. The oil can also be pre-heated, and then used for impregnation, provided that the pre-heating is not done at a level resulting in crosslinking and/or polymerization. Preferably, to choose the optimal result rather than the optimal simplicity of the process, the oil is pre-heated before impregnation in order to reduce viscosity if desired, and, after excess oil is removed, the resulting impregnated wood is heated too, in order to allow for chemical reactions to take place inside the wood.

The impregnated wood is preferably heated to a temperature in the range of from 100°C to 200 °C, preferably of from 120°C to 180°C, at atmospheric pressure. It can be preferred to apply elevated pressure so as to optimally prevent the oil from oozing out. If the oil is pre-heated, this is preferably to a temperature in the range of from 40 °C to 90 °C. A typical duration for the heat treatment is 30 minutes to 4 hours, preferably 1-3 hours, depending on the curing temperature.

As a result of the impregnation and heating steps, the conjugated, preferably multiple conjugated, polyunsaturated fatty acid ends up interlinked with wood (i.e. with wood polymers such as cellulose, hemicellulose, and/or lignin). Without wishing to be bound by theory, the inventors believe that the conjugated, preferably multiple conjugated, polyunsaturated fatty acid, as a result of its relatively high reactivity, upon heating will polymerize. As the polymerization takes place within the impregnated wood, the result is an interpenetrating network (IPN) of the polymerized fatty acid and the lignin network present in wood. This effectively leads to a desirable bonding of the impregnated wood preservation agent with the wood, and thus a fixed hydrophobicity in the wood, so as to render the wood more durable.

In another embodiment, the oil is chemically modified before use so as to introduce chemical functionalities that are capable of chemical binding to wood. Without wishing to be bound by theory, the inventors believe that heating wood impregnated with the reaction product of an oil comprising a conjugated, preferably multiple conjugated, polyunsaturated fatty acid with a dienophile, notably maleic anhydride, will lead to the *in situ* formation of a resin that binds to the wood's hydroxyl group containing constituents.

This embodiment is advantageous *inter alia* in that the improved bonding of the wood preservation agent is believed to be associated with improved wood preservation. Moreover, as compared to the chemical modification disclosed in the art, the presence of conjugated, preferably multiple conjugated, polyunsaturated fatty acids allows for a better, more economical process, e.g. involving a considerably lower reaction temperature and with only a single reaction step required. Thus, other than as described in the aforementioned references (Tjeerdsma et al. and EP 1 174 231), the oil used in the present invention can directly undergo a Diels-Alder cyclo-addition with a dienophile such as maleic anhydride. This reaction occurs at relatively low temperatures (e.g. as of about 100°C), whilst lineseed oil will require a much higher temperature (e.g. as of 200°C) to undergo the first addition reaction. Moreover, these high temperatures lead to the formation of byproducts such as CO₂. Hence, the agent of the invention has a higher degree of purity, is better defined, and can be produced at lower energy costs.

Whilst maleic anhydride is a preferred dienophile, other dienophiles are possible, as well-known to the skilled person, such as itaconic anhydride, citraconic anhydride, and aconitic anhydride. The dienophile is preferably used in at most a stoichiometric amount calculated on the amount of conjugated polyunsaturated fatty acid present in the oil.

The reaction of the dienophile with the oil comprising a conjugated polyunsaturated fatty acid, can be conducted at temperatures well below 200°C, preferably between 100°C and 150°C, more preferably between 110°C and 130°C. The optimal temperature will to some extent depend on the exact combination of diene (i.e. the oil) and dienophile. The eventual choice of reaction circumstances is within the ambit of the skilled person's standard practice.

In accordance with this embodiment of the wood preservation method of the invention, the invention also provides a wood preservation agent obtainable by reacting an oil comprising a conjugated, preferably a multiple conjugated, polyunsaturated fatty acid with a dienophile, under circumstances that allow the formation of a Diels-Alder adduct.

After impregnation with the so-modified oil of the invention, the wood is generally heated to a temperature within the range of from 100°C to 200°C, preferably of from 120°C to 180°C. A typical duration for the heat treatment is 30 minutes to 4 hours, preferably 1-3 hours.

In accordance with the invention, conjugated oils and modified conjugated oils can also be used in combination, in any ratio.

In all of the embodiments of the invention. the impregnation can be conducted by simple immersion of the wood in the liquid preservation agent.

Thus the liquid is used in a generous amount, so as to saturate the wood with the liquid. Any remaining liquid (i.e. not introduced into the wood) can be re-used in a further impregnation.

Impregnation itself is a technique well-known to the skilled person. The skilled person will know how to select, in a specific situation determined by variables such as the type of wood, the desired degree of loading (the desired balance of durability and economics), the appropriate vacuum-presssure sequences in a conventional impregnation vessel.

The viscosity of the liquid wood preservation agent as used in the invention should be low enough to allow proper impregnation. Irrespective of whether the conjugated, preferably multiple conjugated, polyunsaturated fatty acid-based oil in the liquid wood preservation agent of the invention is in the unmodified or modified form, the improved reactivity towards IPN formation and/or bonding with wood allows the viscosity requirements to be more relaxed than in the prior art. With conventional impregnation agents, the viscosity needs to be sufficiently low when impregnating (e.g. by heating an otherwise too viscous oil), and sufficiently high when contained in the wood, so as to avoid leaking out of the agent. The IPN formation and/or bonding with wood will prevent the latter. Nevertheless, for the purposes of impregnation, a relatively low viscosity, below 100 cP, is preferred, more preferably below 80 cP. It will be clear to the skilled person that this refers to the dynamic viscosity, the determination of which is well-known. If needed, the temperature will be adjusted, and/or solvent used, to secure an appropriately low viscosity

Besides the natural oil-based liquid, the wood preservation agent can also contain small quantities of non-aqueous solvent to facilitate penetration into the wood. The choice of non-aqueous solvent is not particularly critical as long as the non-aqueous solvent dissolves the conjugated oil and/or the anhydride functional resin. It is preferred to use a non-aqueous solvent that evaporates easily. The non-aqueous solvent preferably has a low boiling point. It is more preferred to use ketones with a relatively short carbon-chain. It is preferred to have a carbon-chain of between 3 and 7 carbon atoms. It is even more preferred to use methyl isobutyl ketone (4-methyl-2-pentanone).

The liquid wood preservation agent provided by the present invention can further contain additives and adjuvants, such as biocides, anti-oxidants, and oil-soluble catalysts to accelerate the curing of the agent after impregnation.

The method of the invention is generally applicable to any type of wood, particularly to natural, unmodified wood, with the greatest benefits attainable with species such as spruce, pine, beech, maple, birch.

The invention also pertains to the resulting treated, modified wood. As will be apparent from the above, this includes wood comprising an interpenetrating network of wood polymers and a polymerization product obtainable by heating wood impregnated with an oil comprising a conjugated, preferably multiple conjugated, polyunsaturated fatty acid. It also includes wood comprising a bound resin obtainable by heating wood impregnated with the reaction product of an oil comprising a conjugated, preferably multiple conjugated, polyunsaturated fatty acid with a dienophile, notably maleic anhydride.

The invention will be further explained hereinafter with reference to the following, non-limiting Examples.

### Example 1

### Example 1. Preparation of calendula oil - maleic anhydride adduct

450 parts of calendula oil and 50 parts of maleic anhydride were added to a four-necked flask provided with cooler, mechanical stirrer, thermometer (coupled to a heating mantle) and nitrogen inlet. The mixture was stirred in a nitrogen atmosphere at 120°C for 3 hours. The product is a dark yellow-brown oil. Characterisation by GC and NMR spectroscopy showed that maleic anhydride had reacted completely and that a Diels-Alder addition to the calendulate side chains had occurred.

### Example 2. Preparation of tung oil - maleic anhydride adduct

450 parts of tung oil and 50 parts of maleic anhydride were reacted and characterized in the manner described in Example 1. The product is a dark brown oil.

### Example 3 Wood treatment with conjugated oil(s) or derivatives thereof

Between 25 and 30 dried and weighed pine sticks (10 x 1 x 1 cm) were arranged inside a stainless steel reactor (1 liter). A vacuum was applied, and, if the viscosity of the oil was higher than 100 centiPoise, the reactor was heated to a temperature at which the viscosity of the oil was lower than 100 centiPoise. After 30 minutes, the conjugated oil (or derivative thereof), which was first heated to a temperature at which the viscosity of the oil was below 100 centiPoise, was added to the reactor. Subsequently, a pressure of 5 bar was applied to the reactor for 1 hour, while keeping the vessel at the required temperature. Then, the remaining oil was pumped out of the reactor, and pressure was reduced to 1 atmosphere. The vessel was opened, and excess oil was removed from the treated pine sticks by paper tissues. The sticks were transferred to an oven and cured at a temperature between 100 and 200°C for a period long enough to ensure full curing. The cured specimens were weighed in order to determine the amount of oil inside the wood. With calendula oil a weight gain of 27% was determined. With the calendula oil / maleic anhydride adduct from Example 1 also a 27% weight gain was determined. Wood specimens modified by other conjugated oils or derivatives thereof generally had increased weights of between 25 and 35%.

## Claims

1. A method for the preservation of wood, comprising impregnating the wood with a preservation agent in the form of a natural oil-based liquid comprising a polyunsaturated fatty acid, and heating the impregnated wood, wherein the natural oil comprises a multiple conjugated polyunsaturated fatty acid.

2. A method according to claim 1, wherein the natural oil-based liquid comprises the oil itself.

3. A method according to claim 1, wherein the natural oil-based liquid comprises the reaction product of the oil and a dienophile, preferably maleic anhydride.

4. A method according to claim 1 or 2, wherein the oil comprises a triple conjugated polyunsaturated fatty acid.

5. A method according to claim 4, wherein the oil is selected from the group consisting of calendula oil, tung oil, and mixtures thereof.

6. A method according to any one of the preceding claims, wherein the natural oil-based liquid comprises a combination of a natural oil and a reaction product of a natural oil and a dienophile.

7. A wood preservation agent obtainable by reacting an oil comprising a conjugated, preferably a multiple conjugated, polyunsaturated fatty acid with a dienophile, under circumstances that allow the formation of a Diels-Alder adduct.

8. Wood, preferably spruce or pine, comprising a linkage with preservation agent obtainable by heating wood when impregnated with a natural oil-based liquid, wherein the oil comprises a multiple conjugated polyunsaturated fatty acid.

9. Wood according to claim 8, wherein the linkage is in the form of
an interpenetrating network of wood polymers and a polymerization product obtainable by heating wood impregnated with an oil comprising a conjugated, preferably multiple conjugated, polyunsaturated fatty acid in the presence of oxygen.

10. Wood according to claim 8, wherein the linkage is in the form of a bound resin obtainable by heating wood impregnated with the reaction product of an oil comprising a conjugated, preferably multiple conjugated, polyunsaturated fatty acid with a dienophile, preferably maleic anhydride.

11. Wood according to any one of the claims 8 to 10, comprising a linkage as defined in claim 9 and a linkage as defined in claim 10.
